# EUROPEAN PATENT APPLICATION

(11) **EP 3 296 454 A1**
(43) Date of publication of application: **21.03.2018**
(21) Application number: 16792224.4
(22) Date of filing: 13.05.2016
(51) Int. Cl.: D06F 73/02

(54) **CLOTHING TREATMENT APPARATUS**

(30) Priority: 13.05.2015 JP 2015098507
(71) Applicant: Aqua Co., Ltd, Chiyoda-ku Tokyo 100-0005 (JP); Qingdao Haier Washing Machine Co., Ltd., Shandong 266101 (CN)
(72) Inventor: NAKAMOTO, Shigeharu, Tokyo 100-0005 (JP); SUZUKI, Hazime, Tokyo 100-0005 (JP); NAGAI, Takayuki, Tokyo 100-0005 (JP); YAMAUCHI, Tomohiro, Tokyo 100-0005 (JP)
(74) Representative: Ziebig, Marlene
(86) International application number: PCT/CN2016/082113
(87) International publication number: WO 2016/180374

(57) **Abstract**

The present invention provides a clothing treatment apparatus capable of preventing air with ozone from leaking from a closed part of a zipper. A clothing deodorizing apparatus includes: a bag body (10) for accommodating clothes; an ozone supply apparatus for supplying air with ozone into the bag body (10); a throwing inlet (11) arranged on the bag body (10) and opened and closed through a zipper (12); and an end hood (13) for covering a circumference of the closed part of the zipper (12) from an inner surface side of the bag body (10). The end hood (13) is formed by fabrics without air permeability. In addition, the zipper (12) is arranged on the throwing inlet (11) in such a manner that the throwing inlet (11) is locked when a zipper slider (12c) of the zipper (12) is pulled downwards and the throwing inlet (11) is opened when the zipper slider (12c) is pulled upwards.

## Description

### TECHNICAL FIELD

The present invention relates to a clothing treatment apparatus for implementing treatment, such as deodorization and so like, on clothing.

### BACKGROUND

In the past, it is known a clothing refreshing apparatus which includes a storage warehouse capable of hanging the clothes on a hanging rod for storage, and introduces circulating air that has absorbed peculiar smell of clothing into an ozone deodorizer by enabling high-temperature and high-humidity air to perform internal circulation in the storage warehouse, thereby deodorizing the clothing (with reference to a patent literature 1).

Since the clothing refreshing apparatus includes the storage warehouse, an apparatus body is easy to become larger. Therefore, a clothing treatment apparatus, including a bag body for accommodating the clothing and an ozone supply apparatus for supplying air with ozone into the bag body and capable of implementing treatment, such as deodorization and the like, on clothing accommodated in the bag body through ozone, in the manner of easy installing at home without large installation space is considered to be realized.

The clothing treatment apparatus can adopt such a structure that a throwing inlet of the clothing is arranged on the bag body and the throwing inlet is opened and closed by a zipper.

### Existing Technical Literature

### Patent Literature

Patent Literature 1: Japan specifically disclosed No. 04-327900 Bulletin

### SUMMARY

### Problems to be solved by the invention

The above clothing treatment apparatus may have a hidden danger that air with ozone leaks from a closed part of a zipper.

The present invention is a technical solution completed in view of the problem. A purpose of the present invention is to provide a clothing treatment apparatus capable of preventing air with ozone from leaking from a closed part of a zipper.

### Solution for solving the problems

A clothing treatment apparatus in a main embodiment of the present invention includes: a bag body for accommodating clothes; an ozone supply apparatus for supplying air with ozone into the bag body; a throwing inlet arranged on the bag body and opened and closed by a zipper; and a hood for covering a circumference of a closed part of the zipper from an inner surface side of the bag body.

Through the above structure, since the hood can be used for blocking the air with the ozone that will flow to the closed part of the zipper, the air with ozone is difficult to leak from the closed part even if the closed part of the zipper is slightly opened.

In the clothing treatment apparatus of the present embodiment, the hood is formed by fabrics without air permeability.

Through the above structure, the air with the ozone does not pass through the hood, and the air with the ozone is more difficult to leak from the closed part.

Effects and significance of the present invention can be further clarified by describing embodiments below. However, the following embodiments are just example during implementation of the present invention. The present invention is not limited by invention in the following embodiments.

### BRIEF DESCRIPTION OF DRAWINGS

Fig. 1 is a structural diagram illustrating a clothing deodorizing apparatus according to the embodiments;
Fig. 2 is a diagram illustrating a lower central part of a bag body according to the embodiments;
Fig. 3 is a diagram illustrating the lower central part of the bag body according to the embodiments;
Fig. 4 is a structural diagram illustrating an introduction pipe according to the embodiments;
Fig. 5 is a schematic diagram illustrating a flow that the introduction pipe is mounted on the bag body according to the embodiments;
Fig. 6 is a diagram illustrating a lower central part of the bag body in a state of installing an introduction pipe according to the embodiments;
Fig. 7 is a structural diagram illustrating an exhaust and clothing rack retention unit according to the embodiments;
Fig. 8 is a structural diagram illustrating the exhaust and clothing rack retention unit according to the embodiments;
Fig. 9 is a structural diagram illustrating the exhaust and clothing rack retention unit according to the embodiments;
Fig. 10 is a structural diagram illustrating the exhaust and clothing rack retention unit according to the embodiments;
Fig. 11 is a structural diagram illustrating a bag body retention part according to the embodiments;
Fig. 12 is a longitudinal section view illustrating a main part in a state that the bag body is mounted on the bag body retention part according to the embodiments;
Fig. 13 is a structural diagram illustrating an ozone supply apparatus according to the embodiments;
Fig. 14 is a structural diagram illustrating the ozone supply apparatus according to the embodiments;
Fig. 15 is a structural diagram illustrating the ozone supply apparatus according to the embodiments;
Fig. 16 is a structural diagram illustrating the ozone supply apparatus according to the embodiments;
Fig. 17 is a diagram illustrating a connection of an introduction pipe to an ozone supply apparatus and a connection detection made by a pipe detection part according to the embodiments;
Fig. 18 is a diagram illustrating an action that a lock detection part detects that a throwing inlet of a bag body is locked by a zipper according to the embodiments;
Fig. 19 is a diagram illustrating an action that the lock detection part detects that a throwing inlet of a bag body is locked by a zipper according to the embodiments;
Fig. 20 is a structural diagram illustrating a fragrance supply unit according to the embodiments;
Fig. 21 is a structural diagram illustrating the fragrance supply unit according to the embodiments; and
Fig. 22 is a structural diagram illustrating a clothing deodorizing apparatus according to the variation embodiments.

### DETAILED DESCRIPTION

A clothing deodorizing apparatus in an embodiment of a clothing treatment apparatus of the present invention is described below with reference to the drawings.

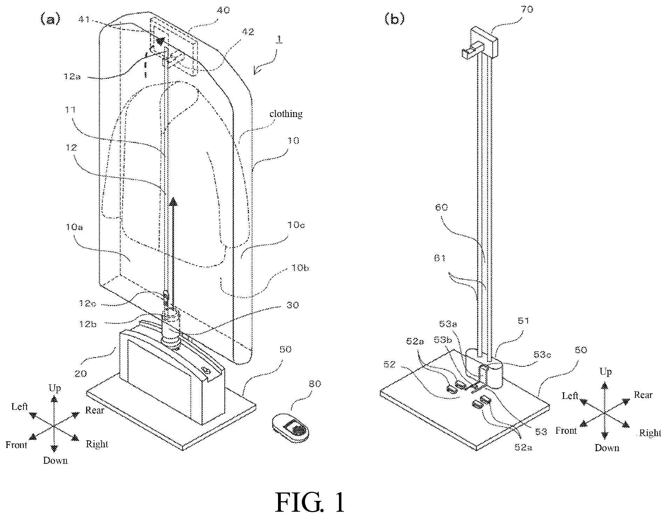

Fig. 1 is a structural diagram illustrating a clothing deodorizing apparatus 1. Fig. 1(a) is a perspective view illustrating the clothing deodorizing apparatus 1. Fig. 1(b) is a perspective view illustrating a base 50, a supporting post 60 and a bag body retention part 70 that form the clothing deodorizing apparatus 1.

By referring to Fig. 1, the clothing deodorizing apparatus 1 includes: a bag body 10, an ozone supply apparatus 20, an induction pope 30, an exhaust and clothing rack retention unit 40, a base 50, a supporting post 60, a bag body retention part 70 and a fragrance supply unit 80.

The bag body 10 accommodates various clothing such as western-style clothes, coats and the like. The bag body 10 is formed in the manner of overlapping a plurality of fabrics having the air impermeability so that tightness is adequate. The bag body 10 has an approximately lengthwise rectangular shape with flat front and rear, and is made of a front fabric 10a forming a front surface, a rear fabric 10b forming a rear surface and side fabrics 10c forming an upper, a lower, a left and a right side surfaces.

An up-down size of the bag body 10 is set as a size capable of accommodating the long clothing such as long shirts, long coats and so like. In addition, a front-rear size of the bag body 10 is set as a size capable of accommodating one piece of clothing. It should be noted that the up-down size of the bag body 10 can also be set as a size incapable of accommodating long clothing, and the front-rear size can also be set as a size capable of accommodating about two or three pieces of clothing arranged in order of the front and rear.

In the front surface of the bag body 10, in an approximate center of a left-right direction, a gap that forms a throwing inlet of the clothing is formed from an upper end to a lower end. A zipper 12 is mounted at the throwing inlet 11. A starting end part 12a and an end part 12b when the zipper 12 performs locking are respectively located on an upper end and a lower end of the bag body 10. A zipper slider 12c of the zipper 12 moves between the starting end part 12a and the end part 12b. When the zipper slider 12c is pulled downwards from the starting end part 12a, the zipper 12 is closed so that the throwing inlet 11 is locked; and when the zipper slider 12c is pulled upwards from the end part 12b, the zipper 12 is opened so that the throwing inlet 11 is opened. In this way, since a pull-down direction of the zipper slider 12c is set as a locking direction of the throwing inlet 11, in a locking state of the throwing inlet 11, self-weight of the zipper slider 12c acts in the locking direction. Therefore, it is different from a case that a pull-up direction of the zipper slider 12c forms the locking direction of the throwing inlet 11, a hidden danger that the end part 12b, i.e., a closed part of the zipper 12, is opened due to the self weight of the zipper slider 12c does not exist.

The ozone supply apparatus 20 performs a deodorization operation for deodorizing the clothing and a fragrance increasing operation for increasing fragrance on the clothing. During the deodorization operation, the ozone supply apparatus 20 supplies air with the ozone to the bag body 10 by performing an action of enabling exhausted air to contain ozone. In addition, when the ozone supply apparatus 20 performs the fragrance increasing operation, the ozone supply apparatus 20 supplies air without the ozone to the bag body 10 by performing an action of enabling the exhausted air without containing the ozone.

The introduction pipe 30 is connected with the bag body 10 and the ozone supply apparatus 20, such that the air exhausted from the ozone supply apparatus 20 is guided into the bag body 10. During the deodorization operation, air with the ozone passes through the introduction pipe 30; and during the fragrance increasing operation, air without the ozone passes through the introduction pipe 30.

An exhaust and clothing rack retention unit 40 is arranged on an upper part of a rear surface of the bag body 10. The exhaust and clothing rack retention unit 40 integrally forms an exhaust part 41 having an ozone removing function and a clothing rack retention part 42 for retaining a clothing rack for hanging the clothing through a resin material. The air with the ozone beneficial for the clothing deodorization is exhausted through the exhaust part 41 from the bag body 10 to an outer side of the bag body 10. When the air passes through the exhaust part 41, the ozone included in the air is removed.

The base 50 is a flat plate with a specified shape, such as a quadrangle. The ozone supply apparatus 20 is carried on the base 50. A supporting part 51 for supporting the supporting post 60 is formed at a rear of the base 50. Moreover, a first fixing part 52 and a second fixing part 53 for fixing the ozone supply apparatus 20 in the manner of enabling the front surface of the ozone supply apparatus 20 to face a direction of the front surface of the base 50 are formed on the base 50. The first fixing part 52 is composed of a plurality of hooked claw parts 52a. The second fixing part 53 includes: an L-shaped elastic rod 53a with one end part supported by the base 50, a bulge 53b formed near the rear slightly relative to one end part of the elastic rod 53a, and a pressing part 53c formed on the other end part of the elastic rod 53a. When the pressing part 53c is pressed downwards, the elastic rod 53a generates elastic deformation and the bulge 53b is contracted into the lower part.

The supporting post 60 is composed of two rods 61. A lower end part of the supporting post 60 is mounted on the supporting part 51, and is erect relative to the base 50. The supporting post 60 may be not composed of two rods 61, but composed of one or more than three rods. In addition, a telescopic mechanism capable of adjusting the height of the supporting post 60 can also be arranged on the supporting post 60.

A bag body retention part 70 is mounted at an upper end of the supporting post 60. The bag body retention part 70 hangs and retains the bag body 10 in such a manner that the bag body 10 cannot move in any direction of front and rear, up and down and left and right.

The fragrance supply unit 80 is used when the fragrance increasing operation is performed through the ozone supply apparatus 20. The fragrance supply unit 80 is detachably mounted on the introduction pipe 30, so that air supplied to the bag body 10 contains fragrant ingredients.

Next, structures of the bag body 10 and the introduction pipe 30 and the exhaust and clothing rack retention unit 40 which are mounted in the bag body 10 are described in detail.

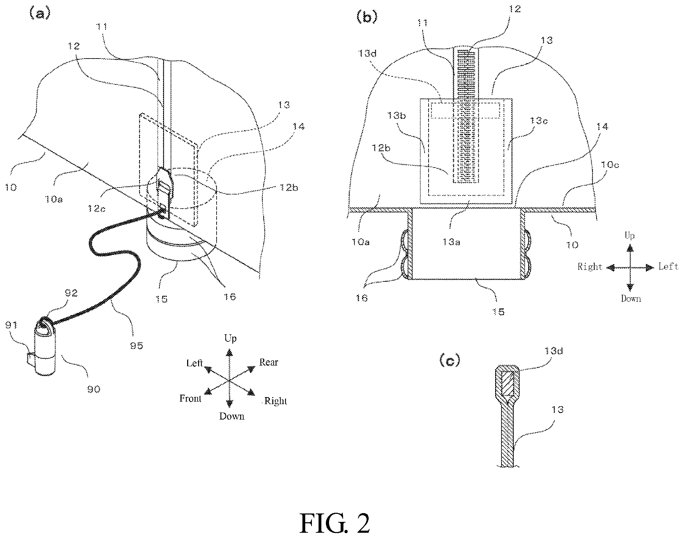

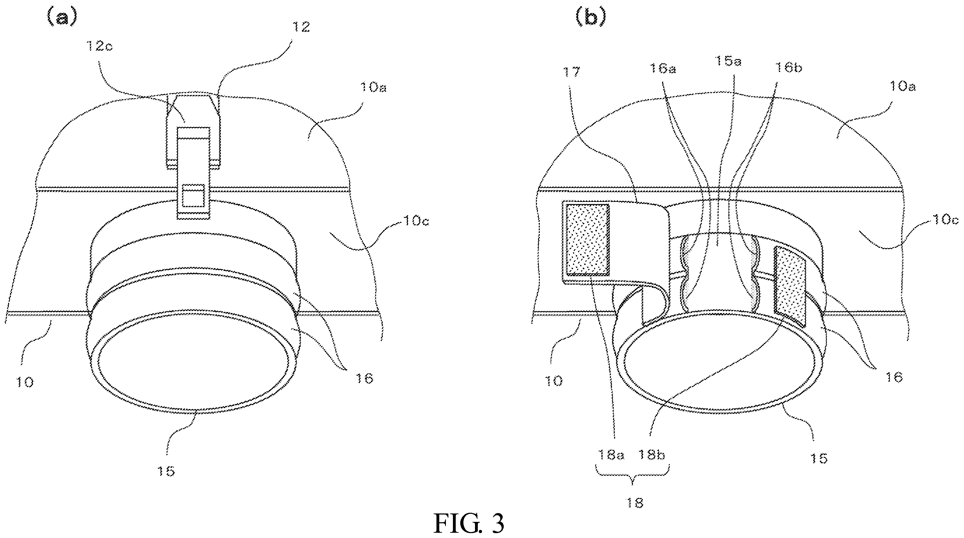

Fig. 2 and Fig. 3 are diagrams illustrating a lower central part of a bag body 10. Fig. 2(a) is a front perspective view, and Fig. 2(b) is a rear section view. Fig. 3(a) is a perspective view during observation from a front lower part. Fig. 3(b) is a perspective view during observation from a rear lower part. Fig. 3(d) shows a side section of an upper part of an end hood 13.

At an inner surface of the front fabric 10a of the bag body 10, the end hood 13 is mounted in the manner of covering the end part 12b of the zipper 12, i.e., the closed part, from an inner side of the bag body 10. The end hood 13 is a rectangle shape formed by fabrics without air permeability, and a lower edge part 13a, a right edge part 13b and a left edge part 13c are tightly fixed to the inner surface of the front fabric 10a through a fixing method such as sewing, bonding and the like. The end hood 13 is equivalent to the hood of the present invention.

When the zipper 12 is not completely closed to the end, the closed part of the zipper 12 is easy to become a slightly open state. As mentioned above, a periphery of the closed part is covered by the end hood. During the deodorization operation, since the air is introduced into the bag body 10, the pressure within the bag body 10 is increased; and since the pressure is increased, the end hood 13 is pushed to the front surface side of the bag body 10 and is easy to become a state close to the inner surface of the front fabric 10a. Therefore, even if the closed part of the zipper 12 is slightly opened, the air with the ozone is difficult to leak from the closed part.

It should be noted that at an upper end part of the end hood 13, filling material 13d made of polyurethane rubber and the like is accommodated in the end hood 13 in a manner that its thickness gets bigger. Thus, the zipper 12 can be prevented from being engaged to the upper end part of the end hood 13 when the zipper 12 is closed.

To detect the locking situation of the throwing inlet 11 through the zipper 12, a detection lock 90 is connected with the zipper slider 12c, and more specifically with a handle of the zipper slider 12c through a connecting rope 95. The detection lock 90 has a cylindrical shape. A protruding part 91 is formed at a lower end part of a circumferential surface of the detection lock 90, and a hanging ring part 92 for fixing the connecting rope 95 is formed at an upper end part of a circumferential surface of the detection lock 90. One end part of the connecting rope 95 is connected with the handle of the zipper slider 12c, and the other end part is connected with the hanging ring part 92. The connecting rope 95 may be a rope with a predetermined length, and for example, is realized by a silk ribbon, a chain, a metal wire and the like.

An air inlet 14 is arranged in the central part of a lower surface of the bag body 10 and a cylindrical part 15 droops from the air inlet 14. The cylindrical part 15 encircles a top end part of the introduction pipe 30 inserted into the air inlet 14. The top end part of the introduction pipe 30 is fixed to the cylindrical part 15.

An approximately loop-shaped belt penetrating parts 16 are arranged nn an outer circumferential surface of the cylindrical part 15 in a vertical parallel mode along a circumferential direction. Both end parts of each belt penetrating part 16 are opened at a rear surface part 15a of the cylindrical part 15 forming the rear surface side of the bag body 10. Each belt penetrating part 16 is used for the following bundling belt to penetrate through.

A belt-shaped shielding part 17 is also arranged on an outer circumferential surface of the cylindrical part 15, to shield a rear surface part 15a. One end of the shielding part 17 is mounted near an open end 16a at one side of upper and lower belt penetrating parts 16. A surface of one side of a hook & loop 18, such as a hook surface 18a, is formed on the other end of the shielding part 17; and a surface of the other side of the hook & loop 18, such as a circular rough surface 18b, is formed near an open end 16b at the other side of the upper and lower belt penetrating parts 16.

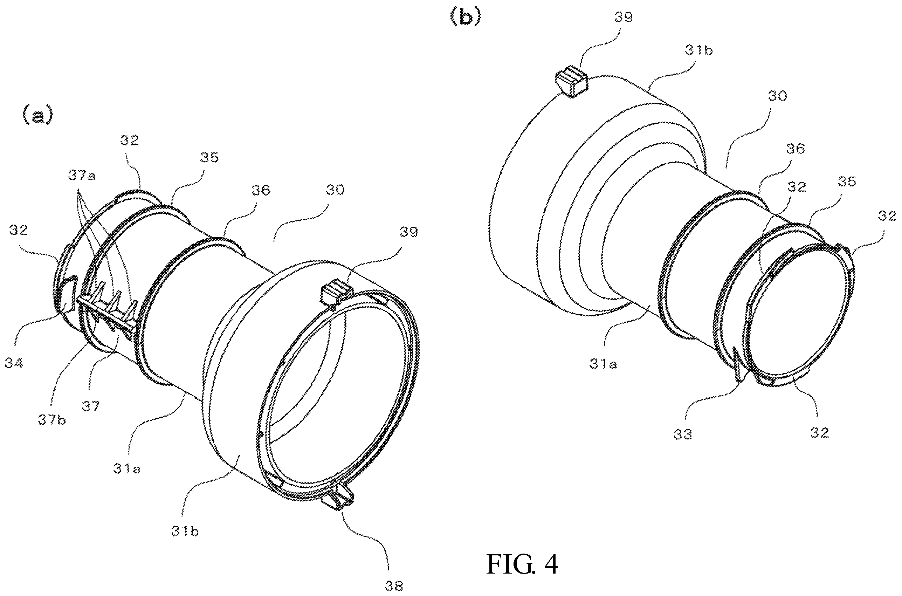

Fig. 4 is a structural diagram illustrating an introduction pipe 30. Fig. 4(a) is a perspective view during observation from a lower end. Fig. 4(b) is a perspective view during observation from an upper end.

The introduction pipe 30 includes a cylindrical main part 31a with a relatively small outer diameter and a cylindrical connecting part 32b with a relatively large outer diameter under the main part 31 a. A boundary part between the main part 31 a and the connecting part 32b has a shape that outlines are gradually centralized together.

A plurality of clamping pieces 32 for fixing the fragrance supply unit 80 are formed at an upper end of the main part 31a. In a position slightly lower than the clamping pieces 32 of the main part 31a and in positions that the introduction pipe 30 faces a front side and a rear side of the bag body 10 in a state of being mounted on the bag body 10, a front flange part 33 and a rear flange part 34 are formed. In a position slightly lower than the front flange parts 33 and the rear flange part 34 of the main part 31a, an annular upper flange part 35 and a lower flange part 36 that have a loop-shaped are formed at a specified interval along an up-down direction. A protruding strip 37 that vertically extends is formed between the upper flange part 35 and the lower flange part 36 and in a position of facing the rear of the bag body 10 in a state of installation on the bag body 10. The protruding strip 37 is formed as an approximate triangular prism shape through triangular ribs 37a vertically parallel and longitudinal ribs 37b for connecting the triangular ribs 37a.

A right claw part 38 and a left claw part 39 are respectively formed at a lower end of the connecting part 31b and in a position facing a right side and a left side of the bag body 10 in a state of installation on the bag body 10.

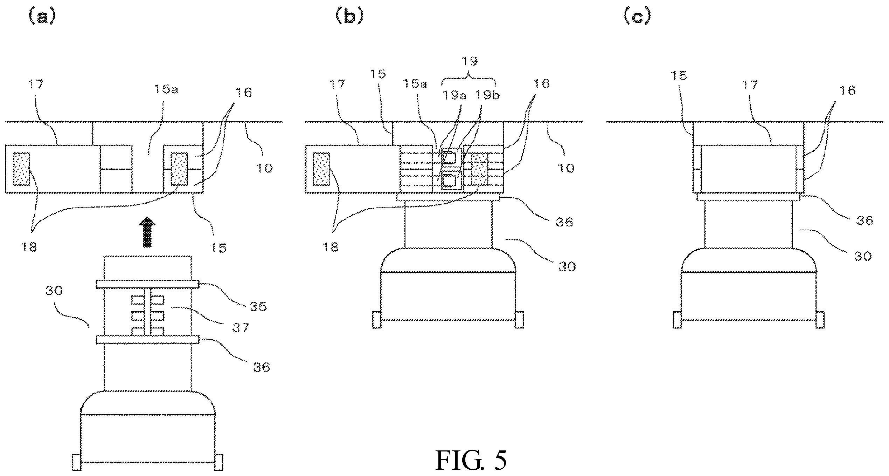

Fig. 5 is a schematic diagram illustrating a flow that the introduction pipe 30 is mounted on a bag body 10.

As shown in Figs. 5(a) and (b), the introduction pipe 30 is inserted into a position close to the lower flange part 36 in the cylindrical part 15 in an orientation that the protruding strip part 37 is located on the rear surface part 15a. As shown in Fig. 5(b), a bundling belt 19 is inserted into a belt penetrating part 16, and wound around the cylindrical part 15. The bundling belt 19 includes a head 19a and a belt part 19b. At a rear surface part 15a, the belt part 19b penetrates through the head 19a, and the cylindrical part 15 is fastened to the inner side through the bundling belt 19. An excessive part of the belt part 19b is cut off. Then, as shown in Fig. 5(c), the other end of the shielding part 17 is fixed to the belt penetrating part 16 through a hook & loop 18. The head 19a is shielded by the shielding part 17. In this way, installation of the introduction pipe 30 on the bag body 10 is completed.

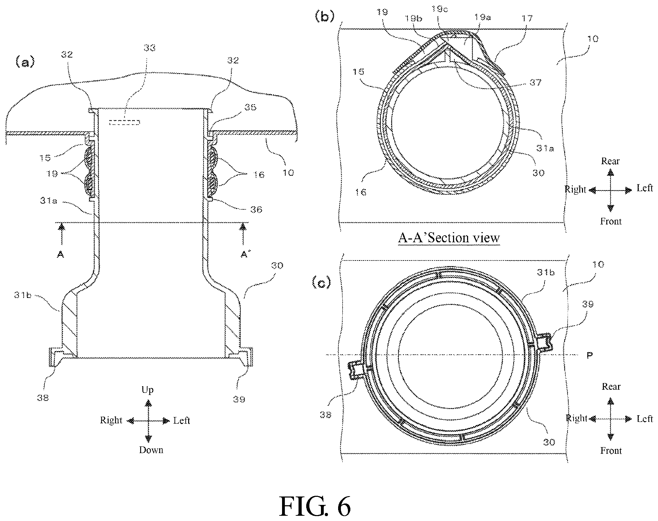

Fig. 6 is a diagram illustrating a lower central part of a bag body 10 in a state of installing the introduction pipe 30. Fig. 6(a) is a rear section view illustrating a lower central part of a bag body 10. Fig. 6(b) is an A-A' section view of Fig. 6(a). Fig. 6(c) is a diagram during observation of a lower central part of a bag body 10 from a lower side.

As shown in Fig. 6(a), in a state that the introduction pipe 30 is mounted on the cylindrical part 15 of the bag body 10, the upper and the lower flange parts 35 and 36 and the bundling belt 19 are clamped along the up-down direction, so that the introduction pipe 30 is fixed to the cylindrical part 15 along the up-down direction. Namely, the introduction pipe 30 cannot move upwards and enter the bag body 10 through the lower flange part 36, and cannot move downwards and separate from the bag body 10 through the upper flange part 35. Moreover, as shown in Fig. 6(b), a combining part 19c of the head 19a and the belt part 19b of the bundling belt 19 is clamped with the protruding strip part 37 of the introduction pipe 30 along the circumferential direction. Thus, the introduction pipe 30 is fixed to the cylindrical part 15 along the circumferential direction. In this way, the introduction pipe 30 is mounted in the manner of not separating from the bag body 10 and also not rotating relative to the bag body 10. In addition, the right claw part 38 and the left claw part 39 of the introduction pipe 30 present a predetermined position relationship relative to the bag body 10. Namely, as shown in Fig. 6(c), the right claw part 38 is in a position slightly forward than a center line P of the front-rear direction of the bag body 10, and the left claw part 39 is in a position slightly backward than the center line P of the front-rear direction of the bag body 10.

As shown in Fig. 6(a), the top part of the introduction pipe 30 is protruded in the manner of being closer to the upper part than the lower surface of the bag body 10. The front flange part 33 and the rear flange part 34 are in positions slightly close to the upper part than the lower surface of the bag body 10.

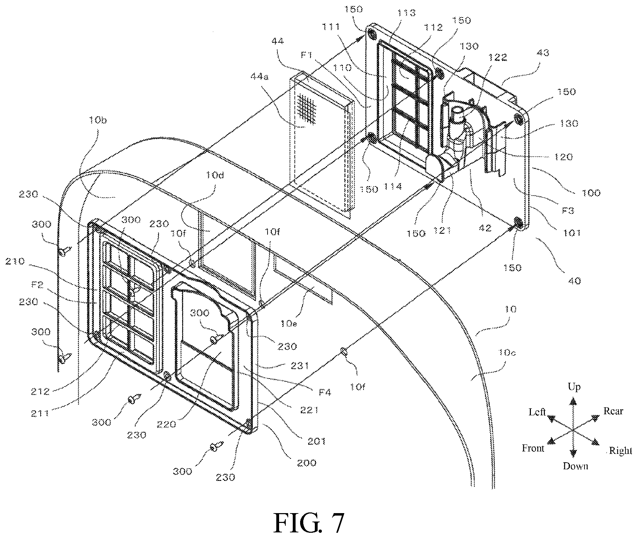

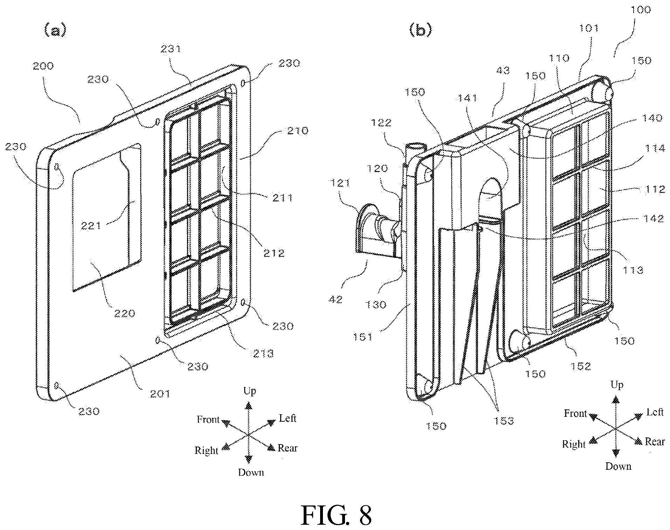

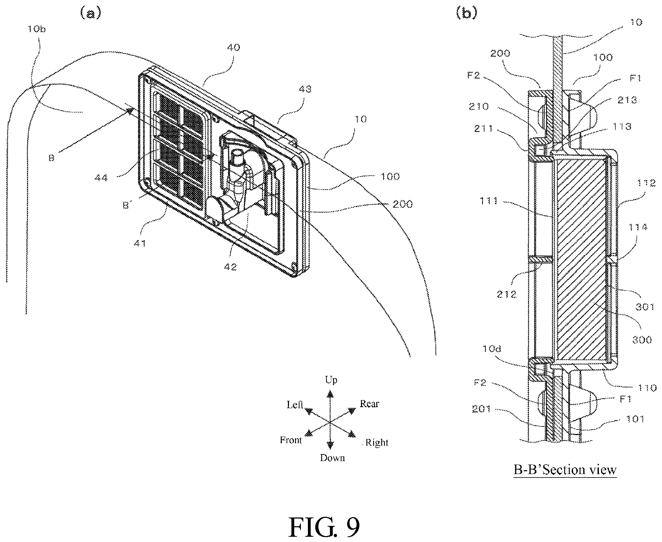

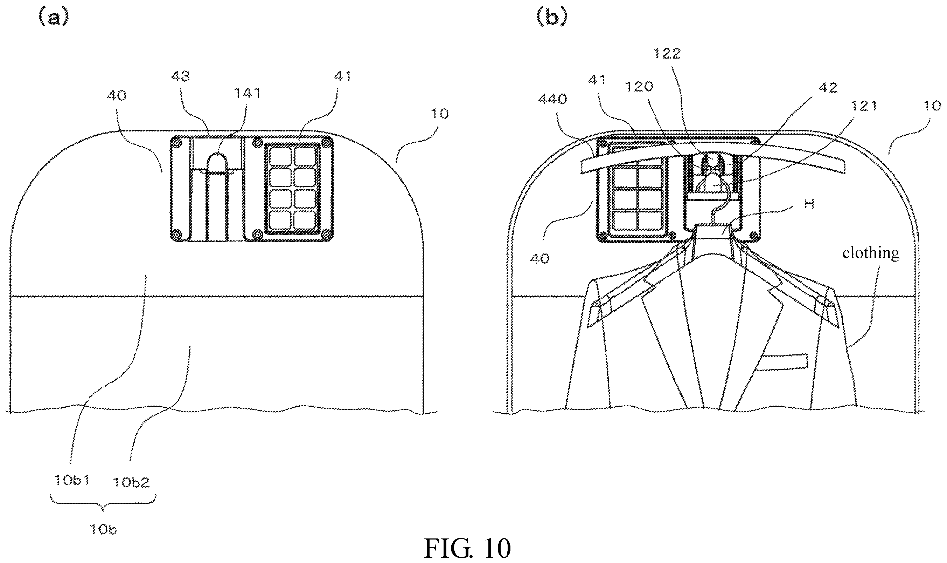

Fig. 7 to Fig. 10 are structural diagrams illustrating the exhaust and clothing rack retention unit 40. Fig. 7 is a perspective view illustrating an exploded exhaust and clothing rack retention unit 40 before being mounted on a bag body 10. Figs. 8(a) and 8(b) are respectively rear perspective views illustrating a front unit 200 and a rear unit 100 forming the exhaust and clothing rack retention unit 40. Fig. 9(a) is a perspective view illustrating the exhaust and clothing rack retention unit 40 mounted on a bag body 10. Fig. 9(b) is a B-B' section view of Fig. 9(a) that an exhaust part 41 is cut off along a horizontal direction. Figs. 10(a) and 10(b) are respectively a rear view and a main view illustrating an upper part of a bag body 10. It should be noted that a front surface of the bag body 10 is not shown in Figs. 7 and 10(b) for convenience, and the bag body 10 is drawn to be transparent in Fig. 9(a).

The exhaust part 41 is formed at a left half part of the exhaust and clothing rack retention unit 40, and the clothing rack retention part 42 is formed at a front side of a right half part of the exhaust and clothing rack retention unit 40. Moreover, an installation part 43 for installing the bag body retention part 70 is formed at a rear side of the right half part of the exhaust and clothing rack retention unit 40.

The exhaust and clothing rack retention unit 40 is formed by combining the rear unit 100 and the front unit 200. The rear unit 100 and the front unit 200 are made of material, such as resin material, harder than the material of the bag body 10. The rear unit 100 and the front unit 200 respectively form constituent elements of an exhaust part 41, a clothing rack retention part 42 and an installation part 43 on a lalongate rectangular rear plate 101 and front plate 201.

The exhaust part 41 includes an exhaust pipe 110 formed on the rear plate 101 and a pipe hood 210 formed on the front plate 201. An ozone removing filter 44 is mounted within the exhaust part 41. The exhaust pipe 110 is formed in the manner of protruding backwards, and is rectangular. The front surface of the exhaust pipe 110 is opened as an inlet/outlet 111 of the ozone removing filter 44, and the rear surface is opened as an air outlet 112. The circumferential edge of the inlet/outlet 111 is enclosed by a guide frame 113 which protrudes forwards. A lattice 114 is formed at the air outlet 112. The pipe hood 210 is formed in the manner of protruding forwards slightly, and includes a rectangular outer frame 211 and a lattice 212 formed within the outer frame 211. A thickness of the outer frame 211 is greater than a thickness of the lattice 212, and a groove 213 is formed at an inner side of the outer frame 211.

The ozone removing filter 44 has a rectangular shape with flat front and rear. The ozone removing filter 44 can use, for example, an activated carbon/catalyst filter formed by transferring activated carbon and a catalyst to base material such as aluminum. A netty hood 44a covers the periphery of the ozone removing filter 44 through a , thus the ozone removing filter 44 cannot be touched by hands. It should be noted that the ozone removing filter 44 can also use other filters with an ozone removing effect, such as a photocatalyst ceramic filter.

The clothing rack retention part 42 includes a first retention part 120 extending from the front surface of the rear plate 101 to the front and a second retention part 121 formed in front of the first retention part 120. A cylindrical inserting port part 122 which protrudes upwards is formed in the first retention part 120. The second retention part 121 has an upward hook shape.

Guide bodies 130 are formed on the front surface of the rear plate 101 and at a left side and a right side of the clothing rack retention part 42. In addition, an opening part 220 through which the clothing rack retention part 42 and the left and the right guide bodies 130 penetrate is formed in the front plate 201. A frame body 221 is formed around the opening part 220.

The installation part 43 includes a body part 140 and an installation hole 141. The body part 140 is formed in a square box shape, and protrudes backwards from the rear surface of the rear plate 101. The installation hole 141 is formed in the rear surface of the body part 140, and extends to the inner part of the clothing rack retention part 42 across the rear plate 101. A notch part 142 is formed on the lower surface of the installation hole 141 in the manner of facing the inner part from an opening end of the installation hole 141.

Screw holes 230 through which screws 300 penetrate are formed in the circumferential position and in positions of the upper end part and the lower end part of the center of the left-right direction of the front plate 201. Moreover, reinforcing ribs 231 which extend all over the entire circumference are formed at an outer circumferential edge of the front surface of the front plate 201.

Installation protrusions 150 for fixing the screws 300 are formed in the circumferential position and in positions of the upper end part and the lower end part of the center of the left-right direction of the rear plate 101. In addition, on the rear surface of the rear plate 101, a first reinforcing rib 151 which encircles the outer edge part of the rear plate 101 and is connected to the right lower end of the body part 140 from the right upper end of the body part 140 is formed at the right side of the installation part 43, and a second rib 152 which encircles the outer edge part of the rear plate 101 and is connected to the left lower end of the body part 140 from the left upper end of the body part 140 is formed at the left side of the installation part 43. Moreover, two third reinforcing ribs 153 which extend from both sides of the installation hole 141 to the lower side are formed on the rear surface of the rear plate 101.

On the upper part of the rear surface of the bag body 10, a first opening part 10d is formed in a position corresponding to the exhaust part 41, and a second opening part 10e is formed in a position corresponding to the clothing rack retention part 42 and the left and the right guide bodies 130. In addition, on the upper part of the rear surface of the bag body 10, insertion holes 10f are formed in positions corresponding to the screw holes 230 of the front plate 201 and the installation protrusions 150 of the rear plate 101.

When the exhaust and clothing rack retention unit 40 is assembled, firstly, the ozone removing filter 44 is accommodated in the exhaust pipe 110 of the rear unit 100. Next, the guide frame 113 is inserted into the first opening part 10d and the left and the right guide bodies 130 are inserted into the second opening part 10e, and then the rear unit 100 is mounted on the upper part of the rear surface of the bag body 10 from the outer side of the bag body 10. Next, the front unit 200 is mounted on the rear unit 100 from the inner side of the bag body 10. Then, the front unit 200 and the rear unit 100 combined by horizontally clamping the upper part of the rear surface of the bag body 10 are fixed by the screw 300. In this way, as shown in Fig. 9(a), the exhaust and clothing rack retention unit 40 is mounted on the upper part of the rear surface of the bag body 10 when being assembled.

Herein, when the rear unit 100 is mounted on the rear surface of the bag body 10, the guide frame 113 and the guide bodies 130 are respectively inserted into the first opening part 10d and the second opening part 10e for guidance. Thus, the rear unit 100 becomes easy to be mounted on the bag body 10, and the assembly of the exhaust and clothing rack retention unit 40 becomes easy.

In addition, as shown in Fig. 9(b), in the exhaust part 41, the top part of the guide frame 113 of the exhaust pipe 110 is embedded into the groove 213 of the outer frame of the pipe hood 210. Thus, since a sealing effect between the exhaust pipe 110 and the pipe hood 210 is enhanced, the air with the ozone, which passes through the exhaust part 41, becomes difficult to leak from the exhaust part 41.

Then, a flat position around the exhaust pipe 110 of the rear plate 101 acts as a rear flange part F1 for covering the circumference of the first opening part 10d from the outer side, and a flat position around the pipe hood 210 of the front plate 201 acts as a front flange part F2 covering the circumference of the first opening part 10d from the inner side. As shown in Fig. 9(b), when the exhaust part 41 is mounted on the first opening part 10d, the first opening part 10d is in a state that its circumference is sealed by the rear flange part F1 and the front flange part F2. Thus, the air with the ozone in the bag body 10 becomes difficult to leak from the first opening part 10d.

Similarly, a flat position around the clothing rack retention part 42 of the rear plate 101 and the left and the right guide bodies 130 acts as a rear flange part F3 for covering the circumference of the second opening part 10e from the outer side, and a flat position around the opening part 220 of the front plate 201 acts as a front flange part F4 for covering the circumference of the second opening part 10e from the inner side. When the clothing rack retention part 42 is mounted on the second opening part 10e, the second opening part 10e is in a state that its circumference is sealed by the rear flange part F3 and the front flange part F4. Thus, the air with the ozone in the bag body 10 becomes difficult to leak from the second opening part 10e.

In a state that an exhaust and clothing rack retention unit 40 is mounted on an upper part of a rear surface of the bag body 10, as shown in Fig. 10(a), the installation hole 141 of the installation part 43 is located in a center of a left-right direction of the bag body 10. In addition, as shown in Fig. 10(b), the clothing rack retention part 42 is arranged in the bag body 10 and located in a central part of the left-right direction of the bag body 10. A hook of the clothing rack H hanging the clothing is hooked on the second retention part 121 of the clothing rack retention part 42, and an upper hood 440 is mounted at an inserting port part 122 of the first retention part 120. The upper hood 440 is formed as a lalongate platy shape slightly bent into an arch. The upper surface of the bag body 10 is strengthened from an inner side through the upper hood 440.

The upper part 10b1 of the rear fabric 10b forming on the upper part of the rear surface the bag body 10 becomes harder than other parts 10b2 by making a thickness become larger than a thickness of other parts 10b2 of the rear fabric 10b or changing the material of the fabric of other parts 10b2. Thus, the exhaust and clothing rack retention unit 40 can be reliably retained through the upper part of the rear surface of the bag body 10.

Next, the structure of the bag body retention part 70 and the installation structure of the bag body 10 about the bag body retention part 70 are described in detail.

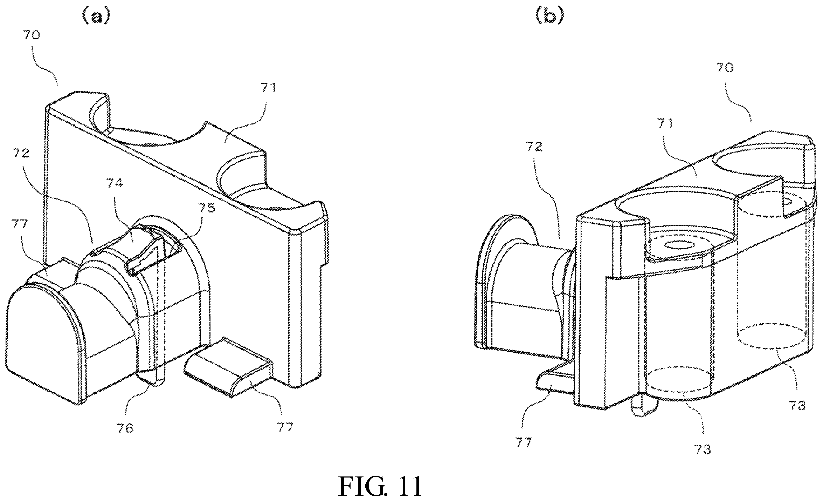

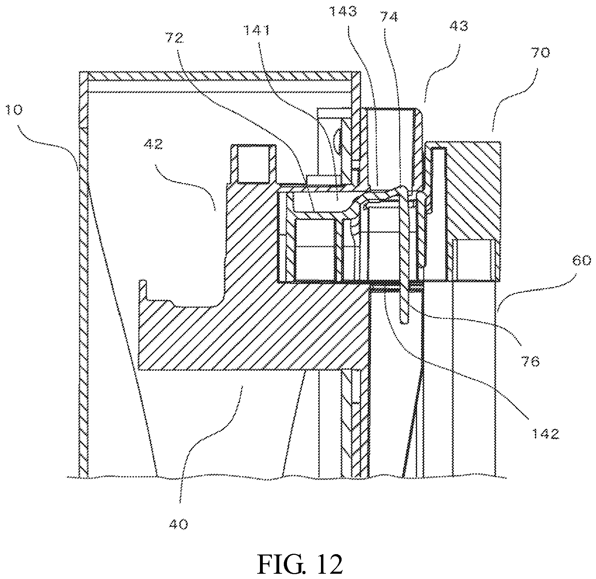

Fig. 11 is a structural diagram illustrating a bag body retention part 70. Fig. 11(a) is a front perspective view, and Fig. 11(b) is a rear perspective view. Fig. 12 is a longitudinal section view illustrating a main part in a state that the bag body 10 is mounted on a bag body retention part 70.

The bag body retention part 70 includes a box-shaped body part 71 and a retention part 72 which extends forwards from the body part 71. Cylindrical insertion holes 73 are formed at a left side and a right side of the body part 71. An upper end part of a rod 61 is inserted into the insertion holes 73.

A clamping claw part 74 is formed on an upper part of a root part of the retention part 72. An opening part 75 is formed around the clamping claw part 74. An inner part of the retention part 72 is hollow, and an operation sheet 76 which droops from the clamping claw part 74 protrudes to the lower part of the retention part 72 from the inner parts of the opening part 75 and the retention part 72. When the operation sheet 76 is pulled to the lower side, the clamping claw part 74 is contracted into the lower side.

The top part of the retention part 72 has an upward hook shape. When the bag body 10 is not mounted on the bag body retention part 70, the clothing rack can be hooked on the top part. Supporting sheets 77 are formed at a lower end of the front surface of the body part 71 and locate on the left side and the right side of the retention part 72.

As shown in Fig. 12, under the condition that the bag body 10 is mounted on the bag body retention part 70, the retention part 72 of the bag body retention part 70 is inserted into the installation hole 141 of the exhaust and clothing rack retention unit 40 by user. During insertion, the operation sheet 76 of the retention part 72 passes through the notch part 142 of the installation hole 141. A clamping hole 143 is formed in the upper part of the inlet part of the installation hole 141. When the retention part 72 is inserted into the installation hole 141 totally, the clamping claw part 74 is clamped with the clamping hole 143. Thus, the retention part 72 is not separated from the installation hole 131. The bag body 10 is fixed in the manner of not moving towards the up-down direction and the left-right direction and not separating forwards relative to the bag body retention part 70. In addition, the lower surface of the installation part 43, although not shown in Fig. 12, is supported by the left and right supporting sheets 77. As shown in Fig. 1(a), in a state of being fixed to the bag body retention part 70, the front surface of the bag body 10 faces the front direction of the base 50.

The operation sheet 76 is pulled to the lower part by user so that the clamping claw part 74 is contracted and the bag body 10 is moved to the front part, thereby removing the bag body 10 from the bag body retention part 70.

Next, a detailed structure of the ozone supply apparatus 20 is described.

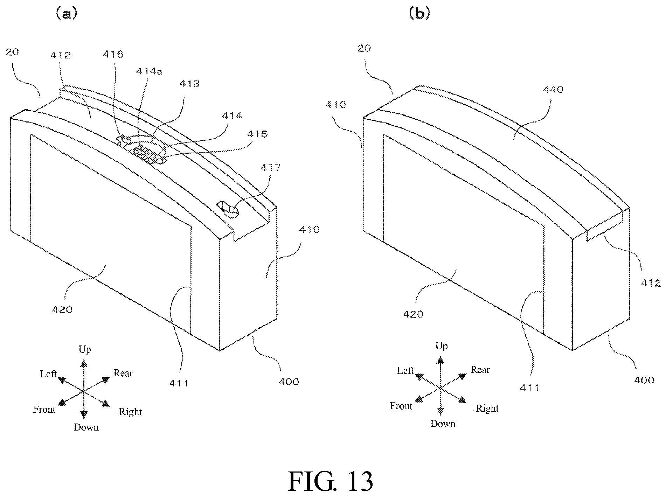

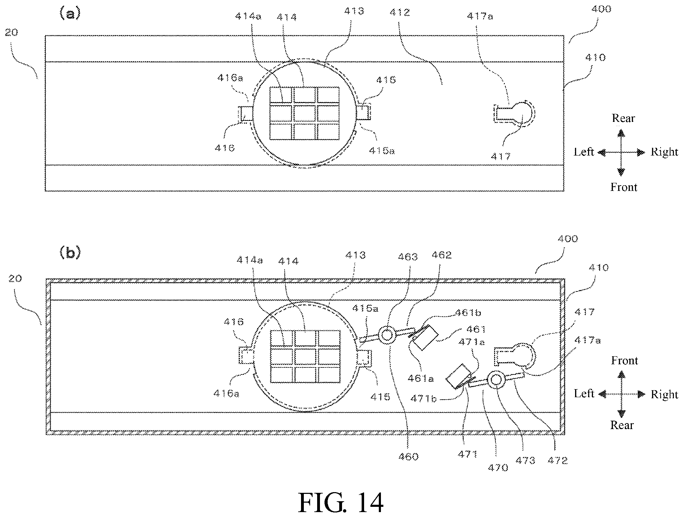

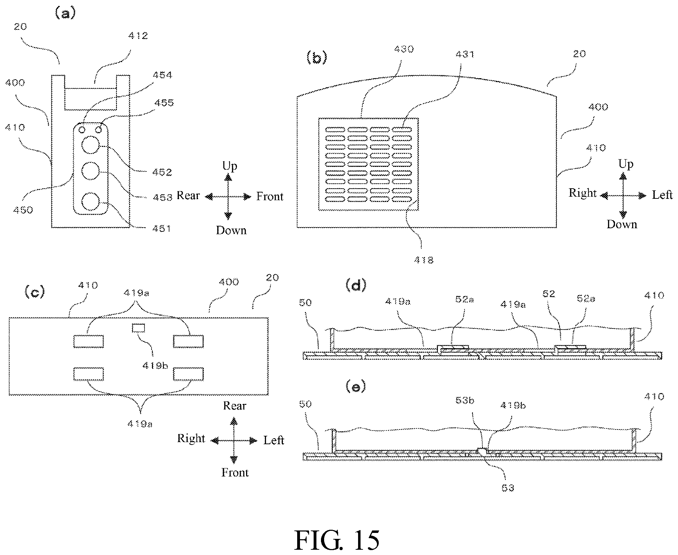

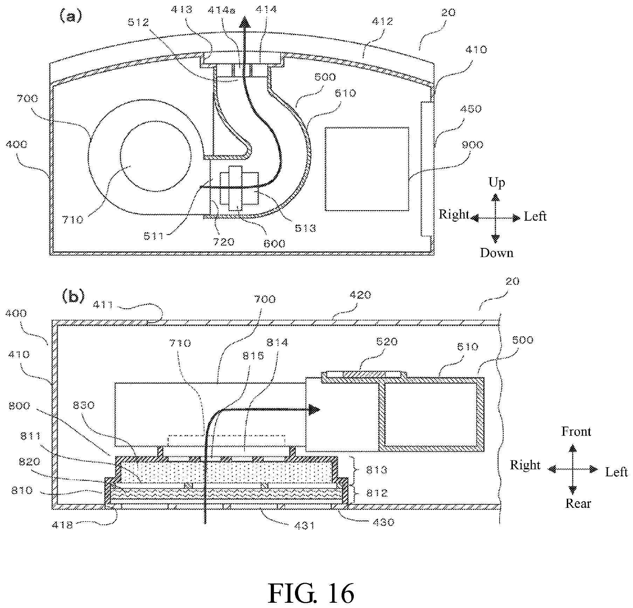

Fig. 13 to Fig. 16 are structural diagrams illustrating an ozone supply apparatus 20. Fig. 13(a) is a perspective view illustrating an ozone supply apparatus 20 in a state without installing an upper hood 440. Fig. 13(b) is a perspective view illustrating an ozone supply apparatus 20 in a state with an upper hood 440. Fig. 14(a) is a top view illustrating an ozone supply apparatus 20. Fig. 14(b) is a cross section view for observing an upper surface of a housing 400 of an ozone supply apparatus 20 from an inner side. Figs. 15(a) to (c) are a left view, a rear view and a bottom view illustrating an ozone supply apparatus 20 respectively. Figs. 15(d) and (e) are side section views illustrating a main part of an ozone supply apparatus 20 in a state of being fixed to the base 50. Fig. 16(a) is a longitudinal section view for observing an ozone supply apparatus 20 from a rear. Fig. 16(b) is a cross section view for overlooking a main part of an ozone supply apparatus 20. It should be noted that a right inserting concave part 415, a left inserting concave part 416, a lock inserting concave part 417, a pipe detection part 460 and a lock detection part 470 are not shown in Fig. 16(a).

The ozone supply apparatus 20 includes a housing 400, a vent pipe 500, an ozone generator 600, a blowing fan 700, an air suction unit 800 and a control unit 900.

The housing 400 includes: a housing body 410, a front hood 420, an air suction hood 430, an upper hood 440 and an operation part 450.As shown in Fig. 13(a), the housing body 410 has a lalongate rectangular shape with an upper surface gently bent. A front surface opening part 411 is formed on a front surface of the housing body 410. The front surface opening part 411 is locked detachably through the front hood 420.

A concave part 412 formed On the upper surface of the housing body 410 has the same shape as the upper hood 440. An inserting port part 413 arranged in the center of the concave part 412 has a circular shape. An exhaust port 414 with a lattice-shaped rib 414a for restricting the flow is formed at the inserting port part 413. A right inserting concave part 415 and a left inserting concave part 416 with shapes corresponding to the shapes of the right claw part 38 and the left claw part 39 of the introduction pipe 30 are formed on the concave part 412 and locate at the left side and the right side of the inserting port part 413. As shown in Figs. 14(a) and (b), a right opening part 415a is formed at a front side of the right inserting concave part 415 in such a manner that the right claw part 38 inserted into the right inserting concave part 415 only moves toward a right turning direction by about an amount of one right claw part 38. Similarly, a left opening part 416a is formed at a rear side of the left inserting concave part 416 in such a manner that the left claw part 39 inserted into the left inserting concave part 416 only moves toward a right turning direction by about an amount of one left claw part 39.

The lock inserting concave part 417 with a shape corresponding to the shape of the detection lock 90 is formed at a right end part of the concave part 412. As shown in Figs. 14(a) and 14(b), an opening part 417a is formed in a side surface of the rear side of the lock inserting concave part 417.

As shown in Fig. 13(b), when the clothing deodorizing apparatus 1 is not used, the upper hood 440 removed from the bag body 10 can be mounted on the concave part 412. Thus, since a retention place of the upper hood 440 removed from the bag body 10 is ensured, the upper hood 440 can be prevented from being lost when not used. In addition, dust can be prevented from entering the housing 400 from the exhaust port 414 and the lock inserting concave part 417 when the clothing deodorizing apparatus is not used.

As shown in Fig. 14(b), the pipe detection part 460 and the lock detection part 470 are arranged at an inner side of the upper surface of the housing body 410. The pipe detection part 460 includes a detection switch 461 and a relay rod 462. The detection switch 461 has a switch part 461a and a rod part 461b for pressing the switch part 461a. The relay rod 462 can be mounted freely rotatably on a rotating shaft 463 formed at the inner side of the upper surface of the housing body 410. One end of the relay rod 462 is located near the right inserting concave part 415, and the other end comes into contact with the detection switch 461. The lock detection part 470 includes a detection switch 471 and a relay rod 472. The detection switch 471 has a switch part 471a and a rod part 471b for pressing the switch part 471a. The relay rod 472 can be mounted freely rotatably on a rotating shaft 473 formed at the inner side of the upper surface of the housing body 410. One end of the relay rod 472 is located near the lock inserting concave part 417, and the other end comes into contact with the detection switch 471.

As shown in Fig. 15(a), the operation part 450 is arranged on a left side of the housing body 410. The operation part 450 includes a power button 451, a deodorization button 452 and a fragrance increasing button 453. The power button 451 is a button for switching on and or off a power supply of the clothing deodorizing apparatus 1. The deodorization button 452 is a button for starting deodorization operation. The fragrance increasing button 453 is a button for starting fragrance increasing operation. In addition, the operation part 450 includes a first informing part 454 and a second informing part 455. The first informing part 454 includes, for example, LED, and an illuminated lamp is used for informing that the introduction pipe 30 is not connected with the ozone supply apparatus 20. The second informing part 455 includes, for example, LED, and an illuminated lamp is used for informing that the throwing inlet 11 of the bag body 10 is not locked.

As shown in Fig. 15(b), an air suction port 418 is formed in a rear surface of the housing body 410. The air suction port 418 is detachably locked through the air suction hood 430. A plurality of air suction holes 431 are formed in the air suction hood 430.

As shown in Fig. 15(c), at a bottom surface of the housing body 410, a first installation hole 419a is formed in a position corresponding to each claw part 52a of the first fixing part 52 of the base 50, and a second installation hole 419b is formed in a position corresponding to the bulge 53b of the second fixing part 53. After a user presses the pressing part 53c of the second fixing part 53 downwards to contract the bulge 53b, when the claw part 52a loads the ozone supply apparatus 20 on the base 50 through the first installation hole 419a so as to transversely slide the ozone supply apparatus 20, as shown in Fig. 15(d), the claw part 52a is clamped with a bottom surface of the housing body 410. Then, when the user stops pressing the pressing part 53c, as shown in Fig. 15(e), the bulge 53b is embedded into the second installation hole 419b. Thus, the ozone supply apparatus 20 is fixed to the base 50 in the manner of not moving in directions of up and down, front and rear and left and right. Therefore, the ozone supply apparatus 20 can be prevented from falling due to a force applied to the ozone supply apparatus 20 when the bag body 10 is inflated because of the air supplied by the ozone supply apparatus 20.

A vent pipe 500, an ozone generator 600, a blowing fan 700, an air suction unit 800 and a control unit 900 are configured within the housing 400.

As shown in Figs. 16(a) and (b), the vent pipe 500 includes a pipe body 510 and a pipe cover 520. An induction port 511 of the pipe body 510 is connected with the exhaust port 720 of the blowing fan 700, and an eduction port 512 is connected with the exhaust port 414. The ozone generator 600 is arranged near the induction port 511 in the pipe body 510. The pipe body 510 has the following shape: the pipe body 510 extends upwards to the eduction port 512 after bending in the manner of extending from the induction port 511 to the left and beginning to go back to the right over a part of the configuration position of the ozone generator 600. Namely, a part of a downstream side of the pipe body 510 forming the ozone generator 600 crawls in an S shape.

The ozone generator 600 is a discharge type ozone generator. Discharge such as corona discharge, silent discharge and the like is generated between a pair of electrodes, and ozone is generated through the air between a pair of electrodes. At a front surface of the pipe body 510, an opening part 513 is formed in a position corresponding to the ozone generator 600. The opening part 513 is locked through the pipe cover 520. The user can remove the front hood 420 and the pipe cover 520, so as to clean the electrodes through the opening part 513 to maintain the ozone generator 600.

The blowing fan 700 is a centrifugal fan, a suction inlet 710 is arranged in its side surface, and an exhaust port 720 is arranged in its circumferential surface. The suction inlet 710 is opposite to the air suction port 418 on the rear surface of the housing 400. The blowing fan 700 obtains the air from the suction inlet 710, and delivers the obtained air to the ozone generator 600 in the vent pipe 500. The blowing fan 700 can also be other fans besides the centrifugal fan, such as an axial flow fan.

As shown in Fig. 16(b), an air suction unit 800 is arranged between the air suction port 418 of the housing 400 and the suction inlet 710 of the blowing fan 700. The air suction unit 800 includes an air suction pipe 810, a dust filter 820 and an ozone removing filter 830.

The air suction pipe 810 is divided into a first filter accommodating part 812 at a side of the air suction port 418 and a second filter accommodating part 813 at a side of the blowing fan 700 through a latticed dividing plate 811. A dust filter 820 is accommodated in the first filter accommodating part 812, and an ozone removing filter 830 is accommodated in the second filter accommodating part 813. The dust filter 820 removes dust included in the air obtained from the air suction port 418. The ozone removing filter 830 removes the ozone included in the air passing through the dust filter 820. The ozone removing filter 830, which is identical with the ozone removing filter 44 of the exhaust and clothing rack retention unit 40, can be an activated carbon/a catalyst filter.

The air suction pipe 810 is provided with a connecting part 814 connected with the suction inlet 710 of the blowing fan 700. The connecting part 814 is connected to the second filter accommodating part 813 through a communication hole 815.

The control unit 900 includes a CPU, a memory and the like to control the ozone generator 600 and the blowing fan 700.

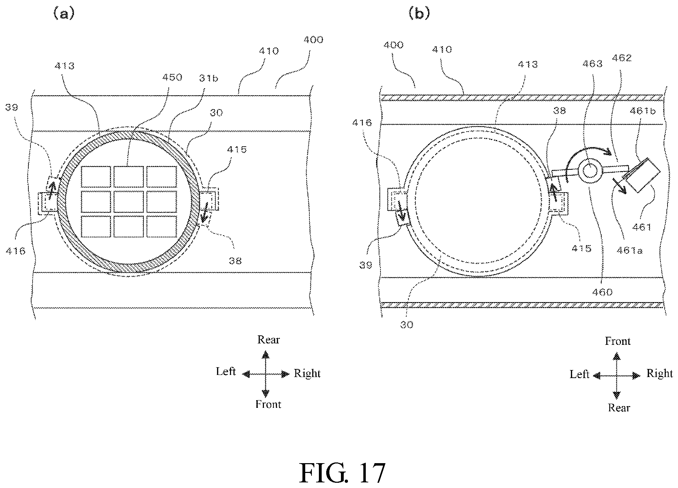

Next, with reference to Fig. 17, a connection of the introduction pipe 30 to the ozone supply apparatus 20 and a connection detection made by the pipe detection part 460 are described.

When the introduction pipe 30 is connected to the ozone supply apparatus 20, as shown in Fig. 17(a), the connecting part 31b of the introduction pipe 30 is inserted into the inserting port part 413 in the manner of respectively inserting the right claw part 38 and the left claw part 39 into the right inserting concave part 415 and the left inserting concave part 416. Then, when the introduction pipe 30 is observed from the upper part and rotates to the right, the right claw part 38 and the left claw part 39 respectively move to the inner side of the upper surface of the housing body 410 through the right opening part 415a and the left opening part 416a, and are clamped with the upper surface of the housing body 410. Thus, the introduction pipe 30 does not fall off upwards.

As described in Fig. 6(c), since the right claw part 38 is in a position slightly forward than a center line P of the front-rear direction of the bag body 10, and the left claw part 39 is in a position slightly backward than the center line P of the front-rear direction of the bag body 10, as shown in Fig. 1(a), the introduction pipe 30 is connected with the ozone supply apparatus 20 in such a manner that the front surface of the bag body 10 faces the front direction of the ozone supply apparatus 20, i.e., the front direction of the base 50. In addition, as mentioned above, the exhaust and clothing rack retention unit 40 is fixed to the bag body retention art 70 in such a manner that the front surface of the bag body 10 faces the front direction of the base 50. Therefore, the bag body 10 is hanged above the ozone supply apparatus 20 in such a state that the upper part and the lower part are hardly distorted. Thus, the clothing can be well accommodated in the bag body 10, and the air with the ozone can be successfully circulated in the bag body 10.

In this way, the introduction pipe 30 is connected with the ozone supply apparatus 20. As shown in Fig. 17(b), when the right claw part 38 moves to the inner side of the upper surface of the housing body 410, one end of the relay rod 462 is pressed by the right claw part 38. The relay rod 462 rotates; a rod part 461b is pressed by the other end of the relay rod 462; and a switch part 461a is pressed by the pressed rod part 461b. Thus, the detection switch 461 detects that the introduction pipe 30 has been mounted on the inserting port part 413.

It should be noted that when the introduction pipe 30 is removed from the inserting port part 413, the rod part 461b rotates the relay rod 462 through elasticity itself, and simultaneously returns to an initial position. Thus, the detection switch 461 detects that the introduction pipe 30 has been removed from the inserting port part 413.

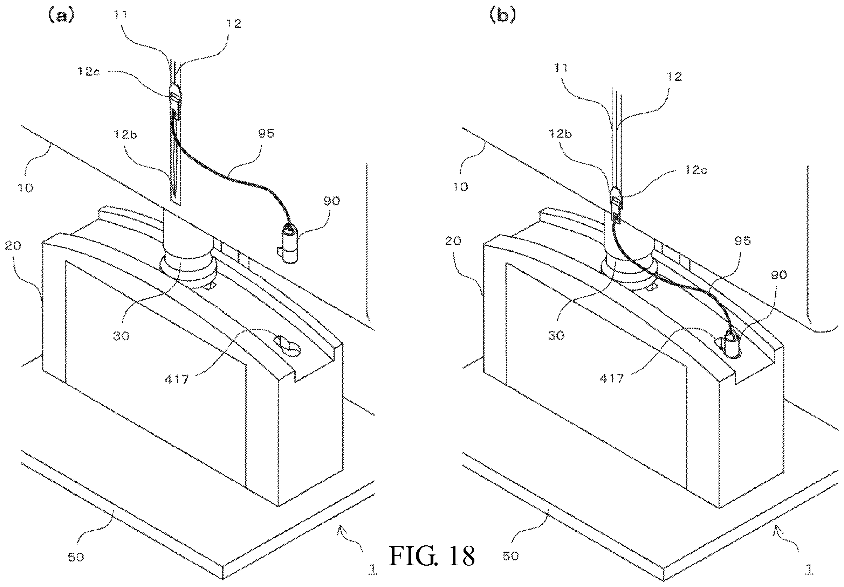

Next, by referring to Fig. 18 to Fig. 19, an action that a lock detection part 470 detects that a throwing inlet 11 of a bag body 10 is locked by a zipper 12 is described.

A connecting rope 95 for connecting the detection lock 90 and the zipper slider 12c has a length that the detection lock 90 arrives at the lock inserting concave part 417 when the zipper 12 is locked to near the end part 12b. Therefore, as shown in Fig. 18(a), when the zipper slider 12c is not located near the end part 12b, the detection lock 90 fails to reach the lock inserting concave part 417 and the user cannot insert the detection lock 90 into the lock inserting concave part 417. Namely, at least in a state that the zipper 12 is completely unzipped, the detection lock 90 fails to reach the lock inserting concave part 417. On the other hand, as shown in Fig. 18(b), in a state that the zipper 12 is completely closed, the detection lock 90 can reach the lock inserting concave part 417 and be inserted into the lock inserting concave part 417.

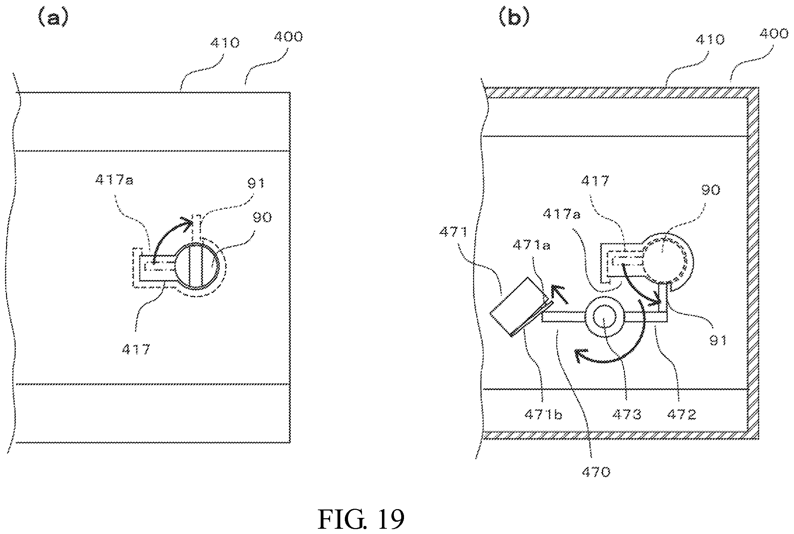

When the user locks the throwing inlet 11 completely through the zipper 12, as shown in Fig. 19(a), the detection lock 90 is inserted into the lock inserting concave part 417 and the inserted detection lock 90 rotates to the right when being observed from the upper part. As shown in Fig. 19(b), the protruding part 91 of the detection lock 90 moves to the inner side of the upper surface of the housing body 410 through the opening part 417a, and one end of the relay rod 472 is pressed through the moved protrusion part 91. The relay rod 472 rotates; the rod part 471b is pressed through the other end of the relay rod 472; and the switch part 471a is pressed by the pressed rod part 471b. Thus, the detection switch 471 detects that the detection lock 90 has been inserted into the lock inserting concave part 417, i.e., detects that the throwing inlet 11 of the bag body 10 has been locked by the zipper 12.

It should be noted that when the detection lock 90 is removed from the lock inserting concave part 417, the rod part 471b rotates the relay rod 472 through elasticity itself, and simultaneously returns to an initial position. Thus, the detection switch 471 detects that the detection lock 90 has been removed from the lock inserting concave part 417.

Next, a detailed structure of the fragrance supply unit 80 is described.

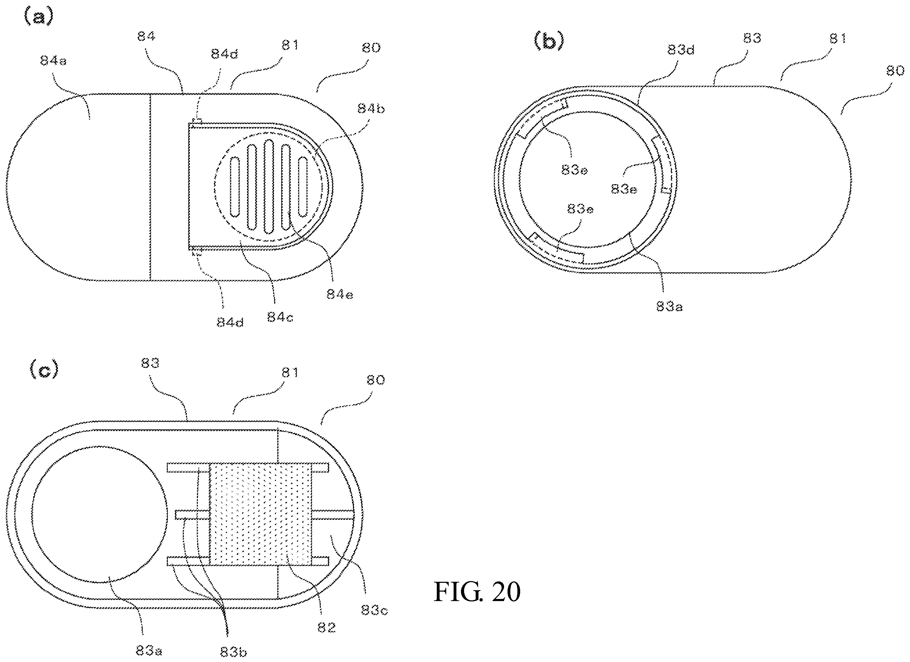

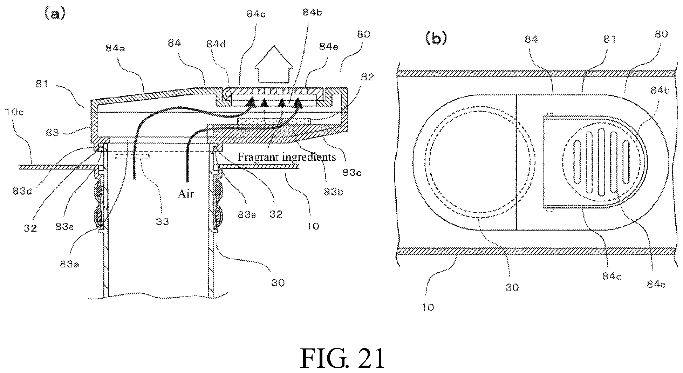

Fig. 20 and Fig. 21 are structural diagrams illustrating a fragrance supply unit 80. Figs. 20(a) and 20(b) are a top view and a bottom view illustrating a fragrance supply unit 80 respectively. Fig. 20(c) is a top view illustrating a fragrance supply unit 80 in a state that an upper box body 84 is removed. Fig. 21 is a longitudinal section view illustrating a central part of a lower part of a bag body 10 in a state that a fragrance supply unit 80 is mounted on an introduction pipe 30. Fig. 21(b) is a cross section view illustrating a central part of a lower part of a bag body 10 in a state that a fragrance supply unit 80 is mounted on an introduction pipe 30.

The fragrance supply unit 80 includes an accommodating box 81 which has an oval box shape when observed from the top, and a fragrant body 82 accommodated in the accommodating box 81. The accommodating box 81 includes a lower box body 83 with an opened upper surface and an upper box body 84 with an opened bottom surface.

At the bottom surface of the lower box body 83, an air suction port 83a is formed at one end of a long edge direction, and a plurality of ribs 83b adjacent to the air suction port 83a and extending along the long edge direction are formed. The fragrant body 82 is loaded above the plurality of ribs 83b. In addition, on the other end, the bottom surface of the lower box body 83 has an inclined surface 83c which becomes higher to the other end. Moreover, at the bottom surface of the lower box body 83, a cylindrical connecting port 83d is formed in the manner of encircling the air suction port 83a. Claw parts 83e are formed in positions that the connecting port 83d corresponds to the clamping pieces 32 of the introduction pipe 30.

The upper box body 84 is mounted on the upper surface of the lower box body 83. The upper surface of the upper box body 84 has an inclined surface 84a which becomes higher to the other end in a position opposite to the air suction port 83a. In addition, an opening part 84b is formed in a position opposite to a plurality of ribs 83b on the upper surface of the upper box body 84. The opening part 84b is covered by a cover part 84c in an openable and closable manner. The cover part 84c rotates by taking a hinging part 84d as a center. A plurality of slit-shaped vent holes 84e are formed in the cover part 84c.

The fragrant body 82 is formed by porous material and the like which can be immersed in a liquid flavoring agent. The user opens the cover part 84c and puts the fragrant body 82 into the accommodating box 81 from the opening part 84b.

When the fragrance supply unit 80 is mounted on the introduction pipe 30, the connecting port 83d is inserted into the top part of the introduction pipe 30 in a state that the claw parts 83e and the clamping pieces 32 are staggered in positions. Then, the fragrance supply unit 80 rotates to overlapping positions of the claw parts 83e and the clamping pieces 32. As shown in Fig. 21(a), the clamping pieces 32 and the claw parts 83e are clamped and the fragrance supply unit 80 does not fall off upwards. It should be noted that the side fabric 10c of the lower surface of the bag body 10 can be prevented from being engaged between the connecting port 83d and the introduction pipe 30 since the front flange part 33 and the rear flange part 34,.

As shown in Fig. 21(b), in a state that the fragrance supply unit 80 is mounted on the introduction pipe 30, the fragrance supply unit 80 is arranged in the bag body 10 in such a manner that a long edge direction of the fragrance supply unit 80 forms a left-right direction of the bag body 10. As mentioned above, since the introduction pipe 30 is fixed to the cylindrical part 15 in the manner of not rotating relative to the bag body 10, the fragrance supply unit 80 correctly mounted on the introduction pipe 30 does not come into contact with the front surface and the rear surface of the bag body 10.

Next, the deodorization operation and the fragrance increasing operation performed on the clothing deodorizing apparatus 1 are described.

Under the condition of performing the deodorization operation, the user accommodates the clothing hanged on the clothing rack H into the bag body 10 hanged on the bag body retention part 70. At this moment, as shown in Fig. 10(b), the user hangs the clothing in the bag body 10 to the second retention part 121 of the clothing rack retention part 42 by using the clothing rack H. In this way, in the bag body 10, the clothing are hanged through the clothing rack retention part 42. The user presses the deodorization button 452 of the operation part 450. When the pipe detection switch 460 detects that the introduction pipe 30 has been mounted on the ozone supply apparatus 20, and the lock detection part 470 detects that the lock 90 has been inserted into the lock inserting concave part 417, i.e., when a condition that the throwing inlet 11 of the bag body 10 is locked by the zipper 12 is detected, the control unit 900 starts the deodorization operation to enable the blowing fan 700 and the ozone generator 600 to operate. When the introduction pipe 30 has not been mounted on the ozone supply apparatus 20, the control unit 900 does not start the deodorization operation and enables the first informing part 454 to illuminate a lamp. In addition, when the throwing inlet 11 is not locked, the control unit 900 does not start the deodorization operation and enables the second informing part 455 to illuminate a lamp.

When the deodorization operation is started, outside air is taken into the air suction pipe 810 through the air suction port 418, and the dust and ozone included in the air can be removed through the dust filter 820 and the ozone removing filter 830 in the air suction pipe 810. The air without dust and ozone is delivered into the vent pipe 500 through the blowing fan 700 (with reference to an arrow in Fig. 16(b)). The air flowing in the vent pipe 500 is mixed with the ozone generated by the ozone generator 600 when passing through the ozone generator 600. In this way, the air with the ozone arrives at the exhaust port 414 through the vent pipe 500 and is exhausted from the exhaust port 414 (with reference to an arrow in Fig. 16(a)).

The air with the ozone exhausted from the ozone supply apparatus 20 is guided into the bag body 10 through the introduction pipe 30. As shown by an arrow in Fig. 1(a), the air with the ozone guided into the bag body 10 is in contact with the clothing in the bag body 10 from bottom to top and simultaneously flows. The clothing is deodorized through a deodorization effect of the ozone included in the air. Herein, although the lower part of the clothing is opened greatly, since the air with the ozone flows from bottom to top in the bag body 10, the air with the ozone is easy to spread over the inner part of the clothing. Thus, comprehensive deodorization can be performed on the outer side and the inner side of the clothing.

In addition, by hanging the bag body 10 to the bag body retention part 70, the clothing is hanged on the clothing rack H, thereby ensuring a clearance between the upper part of the bag body 10 and a shoulder part of the clothing. Thus, since the ozone is also easy to spread over the shoulder part of the clothing, the deodorization effect can be enhanced.

The air with a reduced ozone concentration because of the deodorization for the clothing, as shown by a dotted arrow in Fig. 1(a), is exhausted outside of the bag body 10 through the exhaust part 41 above the bag body 10. The ozone is removed from deodorized air through the ozone removing filter 44 when the deodorized air passes through the exhaust part 41. Thus, the concentration of the ozone in the air exhausted from the bag body 10 is further reduced.

Next, under the condition of performing the fragrance increasing operation, the user accommodates the clothing into the bag body 10 hanged on the bag body retention part 70, and as shown in Fig. 21, in the bag body 10, the fragrance supply unit 80 provided with the fragrant body 82 is mounted on the introduction pipe 30. The user presses the fragrance increasing button 453 of the operation part 450. When the pipe detection switch 460 detects that the introduction pipe 30 has been mounted on the ozone supply apparatus 20, and when the lock detection part 470 detects that the throwing inlet 11 of the bag body 10 has been locked by the zipper 12, the control unit 900 starts the fragrance increasing operation and enables the blowing fan 700 to operate. When the introduction pipe 30 is not mounted on the ozone supply apparatus 20, the control unit 900 does not start the fragrance increasing operation and enables the first informing part 454 to illuminate a lamp. In addition, when the throwing inlet 11 is not locked, the control unit 900 does not start the fragrance increasing operation and enables the second informing part 455 to illuminate a lamp.

When the fragrance increasing operation is started, as shown in Fig. 21(a), the air exhausted from the introduction pipe 30 is introduced into the accommodating box 81 from the air suction port 83a. The introduced air flows upwards after flowing towards the other end along a plurality of ribs 83b. Through the air that passes through the fragrant body 82, the fragrant ingredients included in the fragrant body 82 volatilize, and are mixed into the air. The air with the fragrant ingredients is exhausted into the bag body 10 through the opening part 84b and the vent hole 84e. It should be noted that the air successfully flows in the accommodating box 81 through two inclined surfaces 83c and 84a, arranged on the accommodating box 81.

Similar to the condition of the deodorization operation, the air with the fragrant ingredients flows from bottom to top in the bag body 10. In addition, since air pressure in the bag body 10 is increased, a fragrance increasing effect of the clothing can be enhanced.

### <Effects of Present Embodiment>

The following effect can be realized through the present embodiment.
(1) Since the clothing deodorizing apparatus 1 adopts such a structure that the air with the ozone is supplied by the ozone supply apparatus 20 and the air with the fragrant ingredients is supplied to the bag body 10 for accommodating the clothing to perform deodorization and fragrance increasing operation of the clothing, clothing deodorizing apparatus 1 can be easily arranged in a family without a large arrangement space.
(2) A periphery of the closed part of the zipper 12 is covered by the end hood 13, and the air with the ozone that will flow to the closed part can be blocked by the end hood 13. Thus, during the deodorization operation, even if the closed part of the zipper 12 is slightly opened, the air with the ozone is difficult to leak from the closed part.
(3) Since the end hood 13 is formed by fabrics without air permeability, the air with the ozone does not pass through the end hood 13, and the air with the ozone is more difficult to leak from the closed part.
(4) Since a pull-down direction of the zipper slider 12c is set as the locking direction of the throwing inlet 11, in a locking state of the throwing inlet 11, self-weight of the zipper slider 12c acts in the locking direction. Therefore, a hidden danger that a closed part of the zipper 12 is opened due to the self-weight of the zipper slider 12c does not exist, then the air with the ozone is more difficult to leak from the closed part.

### <Variation Embodiment>

Although embodiments regarding the present invention are described above, the present invention is not limited to the above-mentioned embodiments. In addition, various changes except for the above can also be made to embodiments of the present invention.

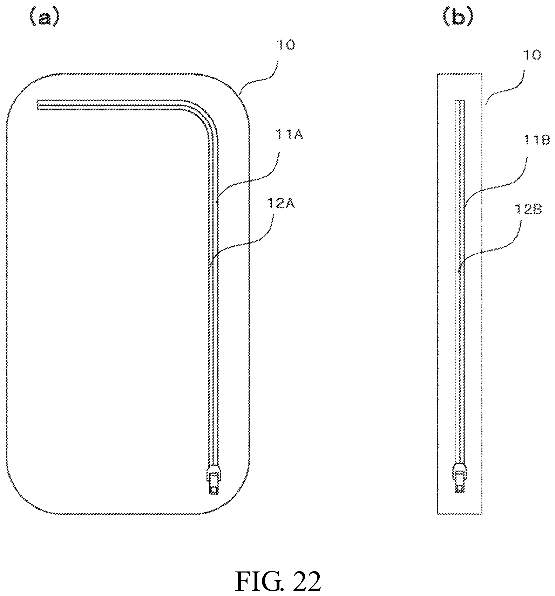

For example, in above embodiments, in the center of the front surface of the bag body 10, the throwing inlet 11 is formed as a gap which straightly extends in an up-down direction. The throwing inlet 11 is opened and closed by the zipper 12, but is not limited to this. For example, as shown in Fig. 22(a), on the front surface of the bag body 10, the throwing inlet 11A is formed as a gap which extends along the upper surface and the side surface of the bag body 10 ; and the throwing inlet 11A is opened and closed by the zipper 12A having a shape corresponding to the throwing inlet 11 A. Or the throwing inlet 11B which straightly extends in the up-down direction like the above embodiment, and the zipper 12B can also be arranged on the left side surface or the right side surface of the bag body 10.

In addition, in above embodiments, the closed direction of the zipper 12 is set as a top-to-bottom direction of the bag body 10. However, the closed direction of the zipper 12 can also be set as a bottom-to-top direction of the bag body 10. In examples shown in Figs. 22(a) and (b), the opened and closed directions may be any direction.

In addition, various changes can be properly made to the embodiments of the present invention within a scope of technical concepts shown in a scope of claims.

### List of reference numerals

10: bag body; 11: throwing inlet; 12: zipper; 12c: zipper slider; 13: end hood (hood); 20: ozone supply apparatus.

## Claims

1. A clothing treatment apparatus, comprising:
a bag body (10) for accommodating clothes;
an ozone supply apparatus (20) for supplying air with ozone into the bag body (10);
a throwing inlet (11) arranged in the bag body (10) and opened and closed by a zipper (12); and
a hood (13) for covering a circumference of a closed part of the zipper (12) from an inner surface side of the bag body (10).

2. The clothing treatment apparatus according to claim 1, wherein
the hood (13) is formed by fabrics without air permeability.
